# FASCICULE DE BREVET EUROPEEN

(11) **EP 2 068 637 B1**
(45) Date de publication et mention de la délivrance du brevet: **23.12.2020**
(21) Numéro de dépôt: 07787515.1
(22) Date de dépôt: 13.07.2007
(51) Int. Cl.: A23C 9/142

(54) **INGREDIENT LAITIER ENRICHI EN LIPIDES POLAIRES ET APPLICATIONS DE CELUI-CI**
MIT POLAREN LIPIDEN ANGEREICHERTER MILCHBESTANDTEIL UND SEINE VERWENDUNGEN
MILK INGREDIENT ENRICHED IN POLAR LIPIDS AND USES THEREOF

(30) Priorité: 17.07.2006 EP 06117336
(43) Date de publication de la demande: 17.06.2009
(73) Titulaire: S. A. Corman, 4834 Goe (BE)
(72) Inventeur: DALEMANS, Daniel, 4040 Herstal (BE); BLECKER, Christophe, 5380 Forville (BE); BODSON, Pascal, 5030 Gembloux (BE); DEROANNE, Claude, 1360 Perwez (BE); PAQUOT, Michel, 5310 Noville sur Mehaigne (BE); DANTHINE, Sabine, 4218 Couthuin (BE)
(74) Mandataire: Pronovem
(86) Numéro de dépôt international: PCT/EP2007/057247
(87) Numéro de publication internationale: WO 2008/009636

(56) Documents cités:
- WO-A-01/78518
- WO-A-02/34062
- WO-A-03/071875
- JP-A- 3 047 192
- JP-A- 2001 275 614
- JP-A- 2005 027 621
- SACHDEVA S ET AL: "RECOVERY OF PHOSPHOLIPIDS FROM BUTTERMILK USING MEMBRANE PROCESSING" KIELER WIRTSCHAFTLICHE FORSCHUNGSBERICHTE, VERLAG TH. MANN, GELSENKIRCHEN, DE, vol. 49, no. 1, 1997, pages 47-68, XP001014232 ISSN: 0023-1347 cité dans la demande

## Description

### Objet de l'invention

La présente invention est relative à un ingrédient laitier enrichi en composants de la membrane des globules gras du lait, c'est-à-dire enrichi en lipides polaires, en particulier en phospholipides et en sphingolipides.

La présente invention concerne également le procédé d'obtention d'un tel ingrédient laitier enrichi en composants de la membrane des globules gras du lait, à partir d'une crème de lait pasteurisée.

Le dernier aspect de la présente invention concerne également l'application avantageuse desdits ingrédients laitiers, à titre de compléments alimentaires, dans des compositions alimentaires, dans des compositions pharmaceutiques ou des compositions cosmétiques.

### Arrière-plan technologique et état de la technique à la base de l'invention

Il est connu depuis de nombreuses années que les produits alimentaires ont des implications sur la santé du consommateur.

En particulier, l'excès de consommation de matières grasses dans l'alimentation humaine peut provoquer l'apparition de pathologies graves engendrées notamment par une augmentation du cholestérol et des triglycérides sanguins, plus spécifiquement le cholestérol.

Pour améliorer l'état de santé du consommateur, on a proposé de nouveaux produits allégés en matières grasses qui malheureusement ne présentent pas les mêmes propriétés organoleptiques que les produits standard.

Il a également été proposé d'utiliser des produits définis comme « alicaments » qui sont constitués par des produits alimentaires présentant un effet thérapeutique ou prophylactique dans le cadre d'un complément au traitement et/ou à la prévention de différentes pathologies (ostéoporose, maladies cardiovasculaires, cancer ...).

De tels ingrédients alimentaires sont par exemple constitués par des sucres, des protéines, des minéraux ou des vitamines susceptibles d'améliorer ou de maintenir la santé de l'homme.

Le document WO02/34062 décrit un procédé pour obtenir un produit enrichi en phospholipides et en sphingolipides par ultrafiltration sur une membrane présentant une valeur de cut-off comprise entre 5000 et 20000 Da et de préférence appauvri ou dépourvu de caséine. Dans ce document, une concentration des sphingolipides (phospholipides, sphingomyéline) par ultra filtration du babeurre lactique de beurrerie, éventuellement déprotéiné ou d'un lactosérum de fromagerie-fromage frais permet d'obtenir un ingrédient laitier enrichi en lipides polaires, dans lequel le pourcentage des phospholipides est inférieur à 3% en poids par rapport au poids de matière sèche de l'ingrédient. Cette demande de brevet décrit également des produits alimentaires ou des suppléments alimentaires comprenant le produit enrichi en phospholipides et en sphingolipides obtenus.

La demande internationale de brevet WO03/071875 décrit un procédé de préparation à partir d'un lactosérum de fromagerie d'un concentré enrichi en lipides polaires laitiers, en particulier un concentré enrichi en sphingolipides laitiers à partir d'un lactosérum de fromagerie. Ce dispositif décrit l'utilisation en parallèle de procédés d'ultrafiltration et de diafiltration au moyen de membranes de 30.000 Da ou de 10.000 Da. Ce procédé nécessite une étape préalable de protéolyse (hydrolyse enzymatique des protéines) de la matière première employée (le lactosérum de fromagerie ultrafiltré). Ce procédé permet d'obtenir un concentré enrichi en sphingolipides (Ultra High Fat Concentration - UHFC) qui peut être ensuite traîté par une phospholipase, afin de purifier les sphingolipides obtenus. Cependant, la concentration en phospholipides dans l'ingrédient laitier obtenu (en poids par rapport au poids de matière sèche de l'ingrédient) est inférieure à 20%.

En outre, le produit concentré obtenu présentera des propriétés organoleptiques détériorées par la présence des composés peptidiques issus de la protéolyse.

De plus, ce document décrit aussi une étape de concentration par ultrafiltration (en parallèle), qui est appliquée après l'hydrolyse enzymatique des protéines. Cette hydrolyse enzymatique permet aux fractions de protéines (peptides) de traverser la membrane d'ultrafiltration et de se séparer de la fraction lipidique qui reste retenue par cette membrane.

SACHDEVA et al. (KIELER WIRTSCHAFTLICHE FORSCHUNGSBERICHTE VERLAG TH. MANN GELSENKIRCHEN, Volume 49 n°1 1997 pp. 47-68) décrit un procédé de récupération des phospholipides à partir du babeurre par un procédé de traitement chimique et physique. Dans ce procédé, la matière première est de la poudre de babeurre doux (sweet cream buttermilk powder)qui est soumise à une coagulation par addition de présure, d'acide citrique ou de culture lactique et de chlorure de calcium, suivie par une séparation et une concentration du sérum par traitement membranaire au moyen d'une ultrafiltration ou d'une microfiltration. Le concentré obtenu est un ingrédient laitier enrichi en phospholipides dans lequel le pourcentage en phospholipides est inférieur à 20% en poids par rapport au poids de la matière sèche de l'ingrédient.

La demande Japonaise JP03/047192 décrit un procédé de fractionnement et de purification d'une fraction de phospholipides dérivés du lait ou d'un produit laitier par l'utilisation d'une étape de chromatographie de partition (ou de partage) liquide-liquide centrifuge.

Les extraits décrits dans cette demande de brevet ont des puretés supérieures à 80% voire 90%, mais ont été obtenus par des extractions aux solvants (éther et acétone). Les rendements sont inconnus puisque la concentration en phospholipides du babeurre initial n'est pas donnée.

L'application de la chromatographie de partition liquide-liquide centrifuge en présence d'autres solvants permet de séparer les phospholipides et donnent les composés purifiés à 97 - 98%, mais les quantités récupérées sont inférieures au milligramme (technique de laboratoire).

La demande de brevet Japonaise 2005 027 621 décrit un procédé de purification de phospholipides à partir de lait ou de produit laitier soumettant ces produits à un traitement de microfiltration par l'utilisation de membranes ayant une taille de pores comprise entre 1 et 2 µm. Ce procédé permet d'obtenir un produit présentant plus de 30% en poids de phospholipides, éventuellement plus de 35% en poids de phospholipides, ce pourcentage étant calculé sur base de la matière sèche de la composition (extrait sec total). En outre, ce document décrit que si la concentration en phospholipides est supérieure à 35% en poids, il est possible d'utiliser la composition de l'invention en tant qu'émulsifiant.

Cette demande de brevet décrit un procédé de concentration de phospholipides par microfiltration d'un lait écrémé (defatted milk) ; un lait écrémé ne contient plus que les petits globules gras dont le rapport « surface/volume » est supérieur, donc qui sont plus riches en phospholipides qui sont les lipides membranaires. Le procédé de microfiltration du lait est traditionnellement employé pour éliminer les microorganismes. L'extrait obtenu est lavé et la partie enrichie en phospholipides est récupérée après rupture de l'émulsion. Cependant, la microfiltration d'un lait entier ne fonctionne pas car elle concentre la matière grasse dans son entièreté (pas d'accroissement du rapport « phospholipides/matière grasse totale ». En outre, le traitement d'une quantité très importante de lait écrémé (qui ne contient que 0.1% de matière grasse et dont seuls les agglomérats de globules de matière grasse dont la taille est supérieure à 2 µm sont récupérés pour obtenir de faibles quantités de phospholipides récupérés (20 tonnes de lait écrémé pour récupérer 200 g d'extrait riche en phospholipides). De plus, le traitement concentre également les polluants microbiologiques qui conduit à une obligation de laver, mais ce lavage n'élimine peut-être pas tous les risques.

La demande de brevet Japonaise JP2001 27 56 14 décrit une composition contenant des phospholipides susceptibles d'avoir une action avantageuse sur la diminution du cholestérol et des triglycérides sanguins et une action inhibitrice sur l'accumulation des lipides neutres dans le foie. En outre, un lien entre les désordres du métabolisme des lipides, le diabète, est également suggéré dans ce document. Les phospholipides utilisés dans ce document sont éventuellement obtenus par des méthodes connues telles que l'extraction par solvant et le fractionnement utilisant différents moyens chromatographiques.

### Buts de l'invention

La présente invention vise à obtenir un ingrédient laitier enrichi en composants lipidiques de la membrane du globule gras du lait, c'est-à-dire enrichi en lipides polaires, en particulier en phospholipides et en sphingolipides et présentant des propriétés physiques particulières mais comportant également et toujours les caractéristiques d'un ingrédient laitier, c'est-à-dire aussi essentiellement dépourvu de protéines hydrolysées, tout en maintenant ou en améliorant les propriétés organoleptiques typiques d'un ingrédient laitier.

La présente invention vise aussi à augmenter les propriétés organoleptiques ou structurelles de compositions alimentaires incorporant ces ingrédients et à apporter une dose journalière adéquate en ces dits lipides polaires, en particulier des sphingolipides, de manière à maintenir ou améliorer un état de santé satisfaisant du consommateur.

Un but particulier de la présente invention vise à fournir des compositions alimentaires qui permettent au consommateur d'obtenir une réduction du taux sanguin de cholestérol et des triglycérides, un effet préventif du cancer, en particulier du cancer du côlon, à renforcer l'immunité et la flore intestinale du consommateur, à obtenir des effets antidiabétiques (traitement et/ou prévention du diabète) et à assurer une protection du foie du consommateur.

Un dernier but de l'invention vise à proposer un procédé (physique) d'obtention de cet ingrédient laitier qui respecte les propriétés organoleptiques de l'ingrédient laitier obtenu et qui soit de conception simple et peu coûteuse et qui présente un rendement amélioré de production de lipides polaires, en particulier des phospholipides et des sphingolipides qui sont particulièrement avantageux, qui s'exprime en un pourcentage en poids élevé par rapport au pourcentage en matière sèche.

### Eléments caractéristiques de l'invention

La présente invention concerne un procédé d'obtention d'un ingrédient laitier enrichi en lipides polaires, en particulier en phospholipides et en sphingolipides, et dont le pourcentage en phospholipides est supérieur à 36%, de préférence de 38% ou de 38,8% en poids par rapport au poids de matière sèche de l'ingrédient, et comprenant les étapes de:
Un chauffage d'une crème pasteurisée de lait (A1) à une température comprise entre 65°C et 70°C pendant une durée comprise entre 5 et 20 minutes,
Une centrifugation d'une crème pasteurisée de lait (A1) en une phase légère d'une crème concentrée (A2) et en une phase lourde de lait écrémé (A3),
Une inversion de la phase légère de crème concentrée (A2) en une émulsion eau dans huile,
une séparation par centrifugation de la phase légère de crème concentrée (A2) en une phase légère de matière grasse de lait (A4) et en une phase lourde de sérum de crème concentrée (A5) dans une proportion entre la phase légère de matière grasse de lait (A4) et la phase lourde de sérum de crème concentrée (A5) qui est comprise entre ¾ et ¼, suivie soit par les étapes de :
   extraction des protéines de la phase lourde de sérum de crème concentrée (A5) par un traitement thermo-calcique comprenant l'ajout de chlorure de calcium, suivi d'un chauffage à une température de 70°C, d'un ajustement du pH du sérum à 5.2 par adjonction d'acide citrique, d'acide phosphorique ou un mélange d'entre eux et un maintien du sérum à cette température pour une durée de 40 minutes et décantation centrifuge pour former un culot de protéines de sérum de crème concentrée (C2) et un surnageant de sérum déprotéiné de crème concentrée (C1),
   une ultrafiltration et une diafiltration avec dilution par de l'eau pour former un rétentat de sérum déprotéiné, ultrafiltré et diafiltré de crème concentrée (C3) et un perméat dilué de sérum déprotéiné de crème concentrée (C4),et
   une récupération de l'ingrédient laitier constitué du rétentat de sérum déprotéiné, ulrafiltré et diafiltré de crème concentrée (C3), dont le pourcentage en phospholipides est supérieur à 36% en poids par rapport au poids de matière sèche de l'ingrédient, ou soit par les étapes de :
      un séchage par atomisation de la phase lourde de sérum de crème concentrée (A5) en une poudre de crème concentrée (A6), combinaison de la phase lourde de sérum de crème concentrée (A5) avec la poudre de crème concentrée (A6) pour obtenir un sérum concentré de crème concentrée (C6),
      une extraction des protéines du sérum concentré de crème concentrée (C6) par un traitement thermocalcique comprenant l'ajout de chlorure de calcium, suivi d'un chauffage à une température de 70°c, d'un ajustement du pH du sérum à 5.2 par adjonction d'acide citrique, d'acide phosphorique ou mélange d'entre eux et un maintien du sérum à cette température pour une durée de 40 minutes et décantation centrifuge pour former un culot de protéines de sérum concentré de crème concentrée (C8) et un surnageant de sérum concentré et déprotéiné de crème concentrée (C7),
      une ultrafiltration et diafiltration avec dilution du surnageant de sérum concentré et deprotéiné de crème concentrée (C7) par de l'eau pour obtenir un rétentat de sérum concentré, déprotéiné, ultrafiltré et diafiltré de crème concentrée (C9) et un perméat dilué de sérum concentré et déprotéiné de crème concentrée (C10), et
      une récupération d'un ingrédient laitier constitué du rétentat de sérum concentré, déprotéiné, ultrafiltré et diafiltré de crème concentrée (C9) et dont le pourcentage en phospholipides est supérieur à 36% en poids par rapport au poids en matière sèche de l'ingrédient.

De préférence, le procédé de l'invention comprend en outre un séchage par atomisation du rétentat de sérum déprotéiné, ultrafiltré et diafiltré de crème concentrée (C3) en une poudre de sérum déprotéiné, ultraflitré et diafiltré de crème concentrée (C5) ou du rétentat de sérum concentré, déprotéiné, ultrafiltré et diafiltré de crème concentrée (C9) en une poudre de sérum concentré, déprotéiné, ultrafiltré et diafiltré de crème concentrée (C11).

### Description détaillée de l'invention

La présente invention est relative à un ingrédient laitier enrichi en composants de la membrane des globules gras du lait, c'est-à-dire enrichi en lipides polaires, en particulier en phospholipides et en sphingolipides, mais également appauvri en protéines, en particulier appauvri en, ou dépourvu de, caséine, tout en assurant que l'ingrédient laitier de l'invention garde les propriétés organoleptiques du produit laitier donc il est issu. On entend par lipides polaires, les lipides portant une tête ou un groupe polaire à l'une de leurs extrémités. Les lipides polaires les plus fréquents sont les phospholipides comprenant des groupes d'acide phosphorique. Parmi ces lipides, on peut citer les phosphoglycérides, tels que le phosphatidyléthanolamine, la phosphatidylcholine, la phosphatidylsérine ou le phosphatidylinositol. Les sphingolipides comportent également une tête polaire, mais aucun groupe glycérol.

On distingue trois sous-classes de sphingolipides : les sphingomyélines, les cérébrosides et les gangliosides. Seules les sphingomyélines comportent un groupe d'acide phosphorique.

Les sphingolipides présents de manière principale dans le lait, sont la sphingomyéline (SPH, ou céramide phosphorylcholine), les glucosides céramides, les lactosyles céramides et les gangliosides. Les phospholipides principalement présents sont la phosphatidylcholine (PC), la phosphatidyléthanolamine (PE), la phosphatidylsérine (PS) et le phosphatidylinositol (PI). Les autres lipides présents dans l'ingrédient laitier de l'invention sont des lipides neutres (triglycérides) et du cholestérol.

Les protéines présentes dans le produit de l'invention sont les protéines laitières, c'est à dire, principalement la caséine, la lactalbumine et la lactoglobuline.

L'objet de l'invention concerne aussi un ingrédient laitier enrichi en ces composants de la membrane des globules gras du lait, en particulier en lipides polaires, c'est-à-dire une composition dont la concentration en ces phospholipides, composants majoritaires des lipides polaires, est supérieure à 36%, 37%, 38% ou 39% en poids de préférence de l'ordre de 39,4% en poids calculée sur le pourcentage en matière sèche de la composition (extrait sec total) et comportant en outre au moins un des éléments choisis parmi le groupe constitué par des protéines, des hydrates de carbone, tels que du lactose, des vitamines solubles dans l'eau, des enzymes, des minéraux et des cendres.

Dans la suite du texte, on regroupe également le lactose et les cendres sous la définition extrait sec dégraissé non protéique.

L'ingrédient laitier de l'invention est caractérisé par sa concentration élevée en lipides polaires, en particulier en phospholipides et en sphingolipides, ainsi que par sa faible concentration relative en ses autres constituants laitiers à savoir, en lactose, en sels minéraux mais aussi en protéines qui sont également de préférence partiellement extraites au cours du procédé d'enrichissement en lipides polaires.

De préférence, le produit de l'invention comprend également un faible pourcentage en protéines, de préférence inférieur à 40%, plus particulièrement inférieur à 30%, de préférence inférieur à 28% (par rapport à la teneur en matière sèche de l'ingrédient). En outre la composition comporte également un certain pourcentage en saccharides (lactose), mais qui peuvent aussi être extraits de la composition.

De manière avantageuse, les protéines de la composition de l'invention sont présentés sous une forme non hydrolysée par l'action d'enzymes protéases et/ou peptidases, afin de ne pas induire d'effets néfastes sur les caractéristiques organoleptiques du produit final (aliments, produits pharmaceutiques,...) obtenu.

L'ingrédient laitier de l'invention peut être présent sous forme liquide ou solide, de préférence obtenu par une évaporation (concentration thermique et séchage) de l'eau présente dans la composition de l'invention, ceci afin d'obtenir des produits plus stables, plus faciles à manipuler ou à doser et moins soumis à une dénaturation biologique.

Un autre aspect de la présente invention concerne le procédé d'obtention de l'ingrédient laitier enrichi en composants de la membrane des globules gras du lait, et comprenant des particules (des globules gras) de lipides ayant de préférence une taille inférieure à 3 µm comprise entre environ 1 µm et environ 2,5 µm, en particulier en lipides polaires, c'est-à-dire en phospholipides et en sphingolipides, de préférence, l'ingrédient laitier de l'invention, en particulier un ingrédient laitier enrichi en lipides polaires c'est-à-dire une composition dont la concentration en phospholipides composés en majorité des lipides polaires est supérieure à 36%, 37%, 38%, 39% en poids de préférence de l'ordre de 39,4% calculé sur le pourcentage en matière sèche de la composition (extrait sec total).

Le procédé l'invention permet d'obtenir à partir d'une crème de lait de préférence pasteurisé, en combinant différentes opérations unitaires qui sont des opérations qui n'altèrent pas les propriétés organoleptiques des produits concentrés obtenus et des co-produits générés, un ingrédient laitier enrichi en ces lipides polaires, c'est-à-dire une composition dont la concentration en phospholipide est supérieure à 36%, 37%, 38% ou 39% en poids de préférence de l'ordre de 39,4% en poids calculé sur le pourcentage en matière sèche de la composition (extrait sec total).

Ce procédé comprend les étapes de centrifugation d'une crème de lait, de préférence pasteurisée en une phase légère d'une crème concentrée et en une phase lourde de lait écrémé, une inversion de la phase légère de crème concentrée en une émulsion eau dans huile, une séparation par centrifugation de la phase légère de crème concentrée en une phase légère de matière grasse de lait et une phase lourde de sérum de crème concentrée suivie, soit par les étapes de:
extraction des protéines de la phase lourde de sérum de crème concentrée par traitement thermocalcique et décantation centrifuge pour former un culot de protéines de sérum de crème concentrée et un surnageant de sérum déprotéiné de crème concentrée,
ultrafiltration et diafiltration du surnageant de sérum déprotéiné de crème concentrée avec dilution par de l'eau pour former un rétentat de sérum déprotéiné, ultrafiltré et diafiltré de crème concentrée et un perméat dilué de sérum déprotéiné de crème concentrée, ou soit par les étapes :
   séchage par atomisation de la phase lourde de sérum de crème concentrée en une poudre de crème concentrée,
   combinaison de la phase lourde de sérum de crème concentrée avec la poudre de crème concentrée pour obtenir un sérum concentré de crème concentrée,
   extraction des protéines par traitement thermocalcique du sérum concentré de crème concentrée et décantation centrifuge pour former un culot de protéines de sérum concentré de crème concentrée et un surnageant de sérum concentré et déprotéiné de crème concentrée,
   ultrafiltration et diafiltration avec dilution par de l'eau du surnageant de sérum concentré et déprotéiné de crème concentré pour former un rétentat de sérum concentré, déprotéiné, ultrafiltré et diafiltré de crème concentrée et un perméat dilué de sérum concentré et déprotéiné de crème concentrée.

Selon une variante d'exécution du procédé de l'invention, celui-ci comprend en outre un séchage par atomisation du rétentat de sérum déprotéiné, ultrafiltré et diafiltré de crème concentrée en une poudre de sérum déprotéiné, ultrafiltré et diafiltré de crème concentrée.

Selon une autre forme d'exécution préférée de l'invention, le procédé de l'invention comporte en outre un séchage par atomisation du rétentat de sérum concentré, déprotéiné, ultrafiltré et diafiltré de crème concentrée en une poudre de sérum concentré déprotéiné, ultrafiltré et diafiltré de crème concentrée.

Avantageusement, le procédé de l'invention comporte également une ou plusieurs étapes (successives) dites de déprotéinisation et aptes à réduire très fortement la concentration des protéines initialement présentes.

De préférence, l'étape de déprotéinisation (coagulation) comporte un traitement thermo-calcique permettant la précipitation de la caséine et le séparation de celle-ci du milieu ; le traitement thermo-calcique comprend l'ajout de chlorure de calcium, suivi d'un traitement thermique à environ 70°C pendant environ 40 minutes, un ajustement du pH à environ 5.2 par ajout d'acide citrique, d'acide phosphorique ou un mélange d'entre eux; la séparation ultérieure des protéines précipitées est réalisée par décantation centrifuge (au moyen d'un séparateur de la phase solide de la phase liquide).

Dans l'étape de traitement thermo-calcique de l'invention, l'addition d'acide citrique est préférée, mais cette étape de coagulation des protéines peut également être obtenue par l'action de la présure et des acides cités ci-dessus.

De préférence, dans le procédé de l'invention, la crème de lait pasteurisée est également soumise à un traitement thermique préalable, par exemple par un chauffage à une température comprise entre environ 60° C et environ 75°C, de préférence à une température comprise entre environ 65°C et environ 70°C, pendant une durée adéquate (environ 5 à environ 20 minutes, c'est-à-dire la durée de la centrifugation continue). Les inventeurs ont observé de manière inattendue qu'une concentration particulièrement élevée des phospholipides dans l'ingrédient laitier est obtenue par le procédé de l'invention, suite au passage des phospholipides dans le sérum. Cet effet est particulièrement important dans les exemples illustrés ci-dessous et n'est pas obtenu lorsqu'on utilise comme matière première une poudre de babeurre doux, tel que décrite dans l'état de la technique. En outre, les inventeurs ont également observé que l'utilisation de certains acidifiants était particulièrement efficace dans l'étape de coagulation, mais que contrairement à l'état de la technique une coagulation par addition de présure adaptée pour obtenir une coagulation (étape de déprotéination) n'est pas efficace pour la formation des produits intermédiaires.

Un dernier aspect de la présente invention concerne une composition pharmaceutique (alicament),une composition cosmétique, une composition alimentaire ou un additif alimentaire comprenant (outre les véhicules pharmaceutiques ou alimentaires adéquats) l'ingrédient laitier enrichi en lipides polaires, en particulier en phospholipides et en sphingolipides selon l'invention, en particulier un ingrédient laitier enrichi en lipides polaires, c'est-à-dire une composition de la concentration en phospholipides et supérieure à 36%, 37%, 38%, 39% voire de l'ordre de 39,4% en poids calculé sur le pourcentage en matière sèche de la composition (extrait sec total). Ladite composition alimentaire ou ledit additif pour composition alimentaire présente des propriétés organoleptiques ou structurelles équivalentes ou améliorées par rapport à des produits alimentaires standards, préparés sans cet ingrédient. De préférence cette composition ou cet additif alimentaire est choisi parmi le groupe constitué par les crèmes, les yoghourts solides, les yogourts liquides, les fromages, les fromages à tartiner, les desserts laitiers, les soupes, les produits de boulangeries et de pâtisserie, en particulier les brioches et les pains au lait.

La composition pharmaceutique comprendra un véhicule pharmaceutique adéquat et ledit ingrédient (à titre de principe actif) en une proportion adéquate pour induire un effet thérapeutique ou préventif de certaines pathologies, (en particulier celles décrites dans les exemples).

La présente invention sera décrite de manière plus détaillée dans les exemples d'exécution ci-dessous présentés à titre d'illustrations non limitatives de l'invention en référence aux figures annexées.

### Description des figures

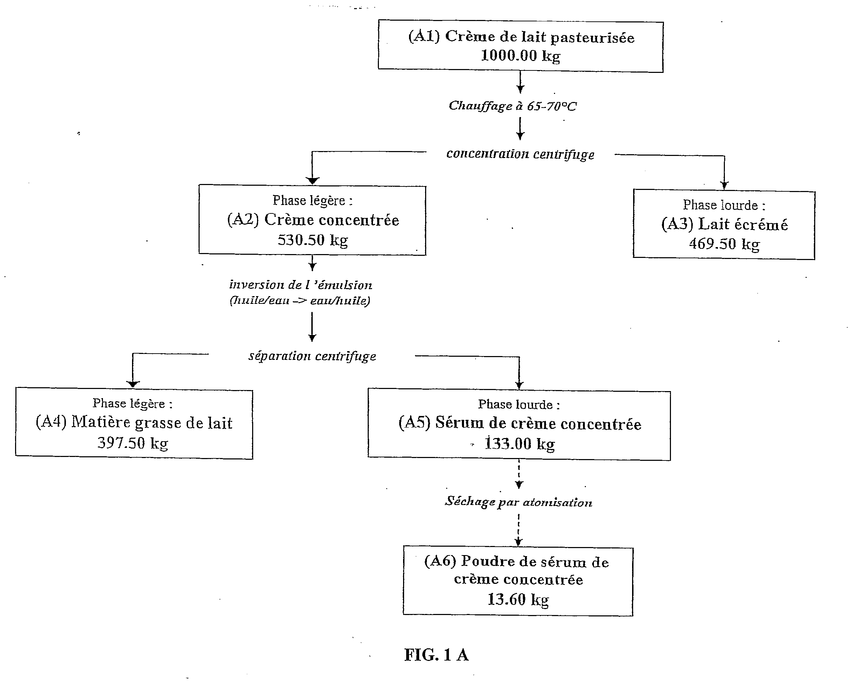

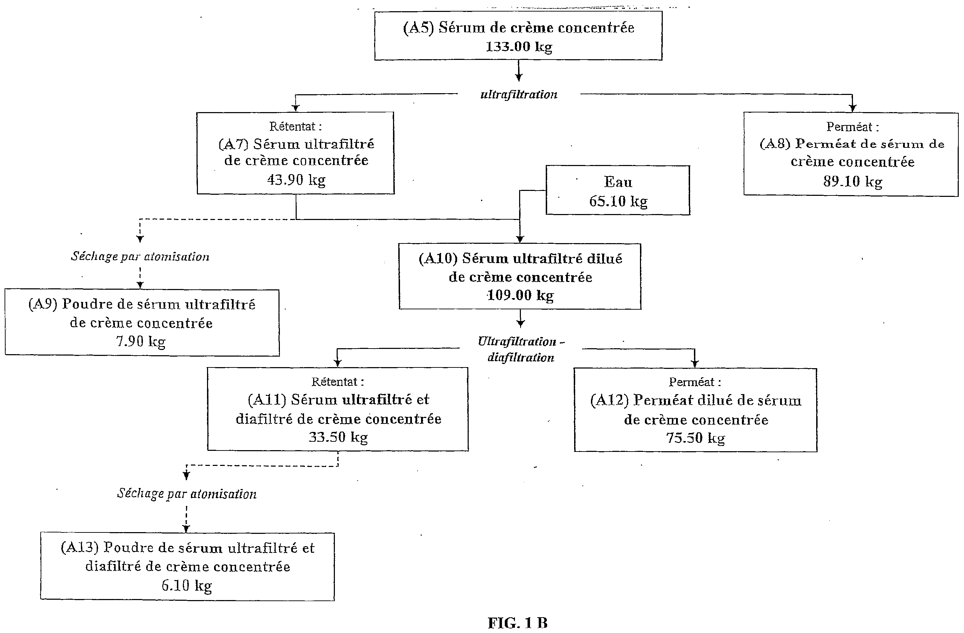

Les figures 1a et 1b représentent, de manière schématique, les différentes étapes du procédé de préparation de l'ingrédient laitier de l'invention à partir de crème de lait pasteurisée. Cet ingrédient est dénommé sérum de crème concentrée (figure la) et est avantageusement concentré par un traitement d'ultrafiltration / diafiltration (figure 1b).

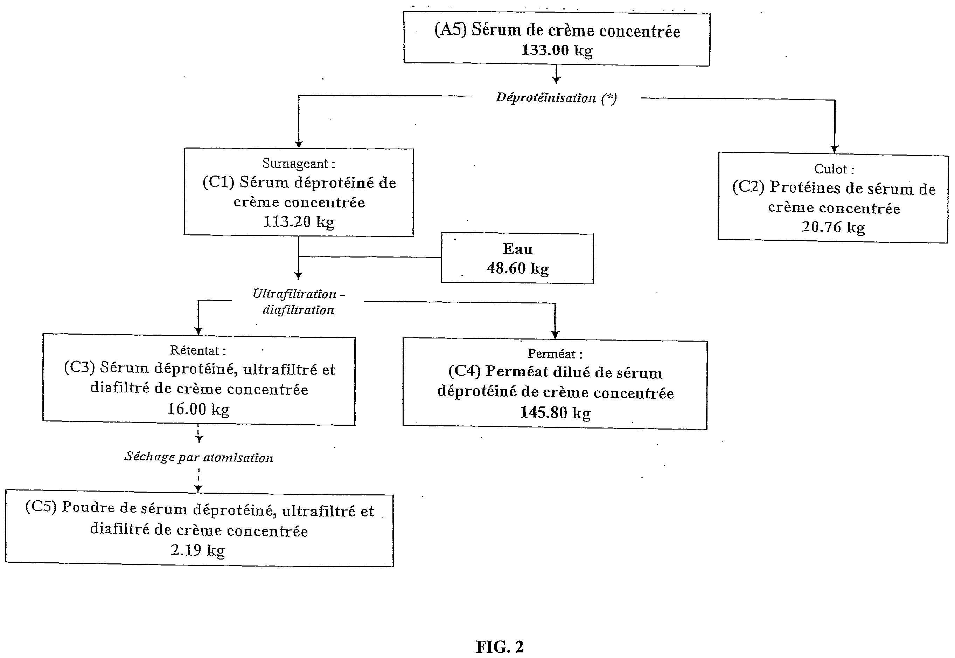

La figure 2 représente, de manière schématique, les étapes du procédé d'obtention de l'ingrédient laitier de l'invention à partir d'un sérum de crème concentrée obtenu par les traitements décrits à la figure la. Une étape de déprotéinisation et une étape de concentration par ultrafiltration et diafiltration permettent d'obtenir un sérum déprotéiné, ultrafiltré et diafiltré de crème concentrée.

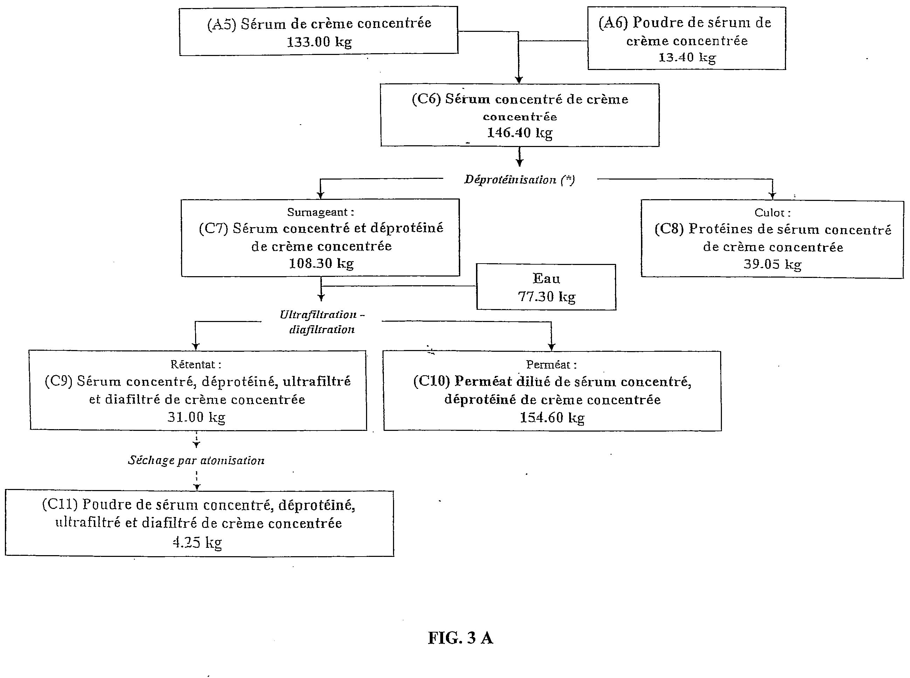

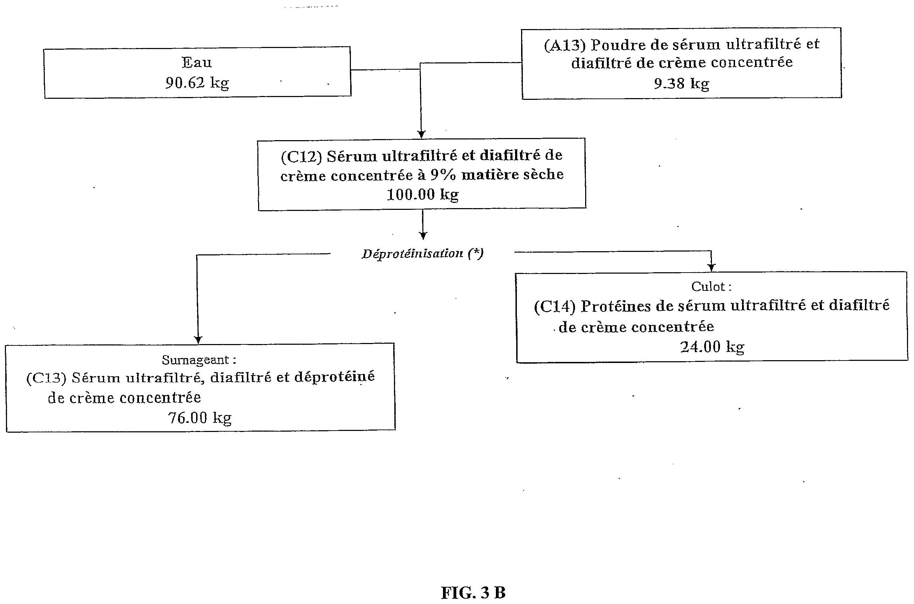

Les figure 3a et 3b représentent, de manière schématique, le même procédé que celui décrit à la figure 2 mais réalisé au départ d'un sérum de crème concentrée enrichi en extrait sec total ; ce procédé donne également un sérum concentré déprotéiné, ultrafiltré et diafiltré de crème concentrée, identique à celui obtenu par les traitements décrits à la figure 2, sans réduction du facteur de concentration des lipides polaires.

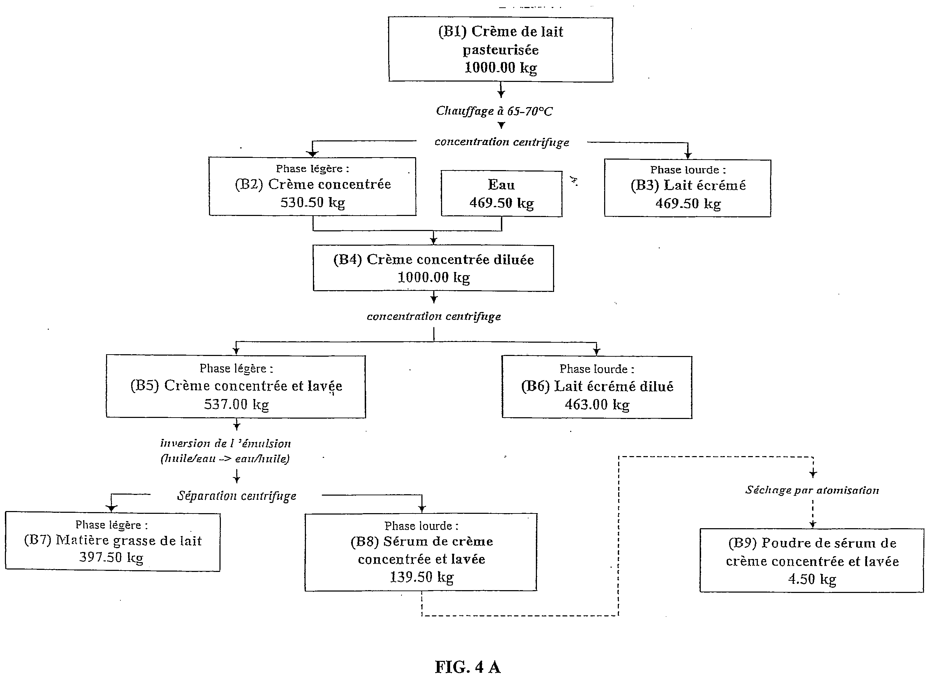

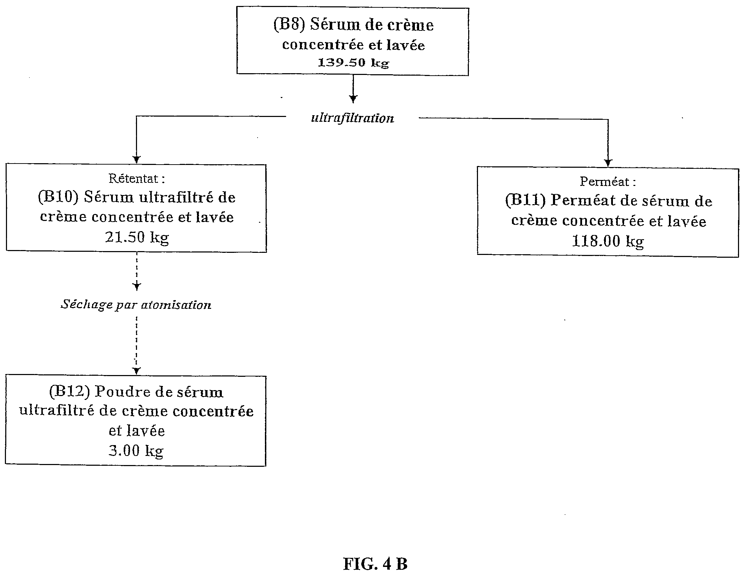

Les figures 4a et 4b représentent, de manière schématique, les différentes étapes du procédé d'obtention de l'ingrédient laitier de l'invention à partir d'une crème de lait concentrée et diluée à l'eau. Cet ingrédient est dénommé sérum de crème concentrée et lavée (figure 4a) et est avantageusement concentré par un traitement d'ultrafiltration et éventuellement de diafiltration (figure 4b).

### Exemple 1

Tel que représenté à la figure 1a, il est possible d'obtenir de manière avantageuse l'ingrédient laitier de l'invention à partir d'une crème de lait de préférence pasteurisée (A1).

Environ 1000 kilos de crème de lait pasteurisée (A1) sont chauffés à une température comprise idéalement entre environ 65°C et environ 70°C (pendant toute l'étape de centrifugation qui se réalise en continu ; cette durée est de l'ordre de 5 à 20 minutes).

La crème de lait ainsi chauffée est soumise à une première centrifugation qui permet de concentrer une phase légère (A2) de crème concentrée et une phase lourde (A3) de lait écrémé. La proportion de la phase légère et de la phase lourde est essentiellement équivalente (environ 530 kilos de la phase légère (A2) et environ 470 kilos d'une phase lourde (A3)).

La phase légère constituée de crème concentrée (A2) sous forme d'une émulsion « huile dans eau » est ensuite transformée en une émulsion « eau dans huile » par une étape dite d'inversion de l'émulsion ; cette étape est suivie d'une deuxième centrifugation permettant de re-concentrer la phase légère de crème (A2) en une nouvelle phase légère (A4) et une nouvelle phase lourde (A5).

La phase dite légère (A4) comprend essentiellement les matières grasses du lait ; la phase lourde constituant un sérum de crème concentrée (A5).

La proportion entre cette phase légère (A4) et cette phase lourde (A5) est d'environ 3/4 - 1/4. (environ 397 kilos de phase légère de matières grasses de lait (A4) et environ 133 kilos de phase lourde (sérum de crème concentrée (A5)).

Ensuite, la phase lourde (A5) est soumise de manière avantageuse à une étape de séchage fournissant un sérum de crème concentrée sous une forme solide (A6) (poudre de sérum de crème concentrée) pour un poids d'environ une quatorze kilos (13,6 kilos).

Selon une première variante d'exécution de l'invention (figure 1b), on récupère et traite ladite phase lourde constituée de sérum de crème concentrée (A5) par une étape supplémentaire comprenant une ultrafiltration permettant de récupérer à la fois un rétentat et un perméat.

Le rétentat est constitué d'un sérum ultrafiltré de crème concentrée (A7) et le perméat est constitué d'un perméat de sérum de crème concentrée (A8). La proportion entre le rétentat et le perméat est d'environ 1/3 - 2/3 (environ 44 kilos de rétentat et environ 89 kilos de perméat).

Selon une alternative de l'invention, il est également possible d'effectuer un séchage direct du sérum ultrafiltré de crème concentrée (A7) pour obtenir un produit solide : une poudre de sérum ultrafiltré de crème concentrée (A9). La quantité de poudre obtenue est d'environ 8 kilos (7,90 kilos).

Selon une autre forme d'exécution préférée de l'invention, le sérum ultrafiltré de crème concentrée (A7) est dilué avec de l'eau, pour former un sérum ultrafiltré dilué de crème concentrée (A10) qui est à nouveau concentré par ultrafiltration (diafiltration).

Cette dilution s'effectue par environ 65 kilos d'eau (càd. 65,10 kg) ajoutée à environ 44 kilos (43,90 kg) de sérum ultrafiltré de crème concentrée (A7) pour former une solution d'un sérum ultrafiltré dilué de crème concentrée (A10) d'un poids total de 109 kilos.

Le sérum ultrafiltré dilué de crème concentrée (A10) est soumis à une seconde étape d'ultrafiltration (diafiltration), de manière à obtenir un nouveau rétentat (A11) et un nouveau perméat (A12), le rétentat étant constitué d'un sérum ultrafiltré et diafiltré de crème concentrée (A11) et le perméat étant constitué d'un perméat dilué de sérum de crème concentrée (A12).

La proportion entre le rétentat (A11) et le perméat (A12) est d'environ 30/70 (35,50 kilos de rétentat (A11) et 75,50 kilos de perméat (A12)).

Le rétentat (A11) peut être à nouveau soumis à une étape de séchage pour obtenir un produit solide : une poudre de sérum ultrafiltré et diafiltré de crème concentrée (A13). Le poids total de cette poudre est d'environ 6 kilos (6,10 kilos).

Le tableau 1 présente les caractéristiques de composition des différents produits obtenus après les différentes étapes réalisée selon le procédé décrit ci-dessus.

Le tableau 1 présente, en pourcentage en poids, les teneurs en eau, en matière grasse totale, en phospholipides (majeure partie des lipides polaires, compris dans la matière grasse totale), en extrait sec dégraissé et les constituants de cet extrait sec dégraissé à savoir, protéines, lactose et cendres.

On observe que le sérum de crème concentrée (A5) soumis aux étapes supplémentaires d'ultrafiltration et de diafiltration est enrichi en composants de la membrane des globules gras du lait, c'est-à-dire en lipides polaires (phospholipides), ainsi qu'en lipides totaux (dont les lipides neutres) et en protéines ; Cet enrichissement se produit essentiellement au détriment des teneurs en lactose surtout et en cendres dans une moindre mesure.

Les produits obtenus sont des ingrédients laitiers riches en lipides polaires. Ces ingrédients laitiers contiennent respectivement plus de 7%, plus de 11% et plus de 14% de phospholipides laitiers, ces pourcentages étant exprimés par rapport à la matière sèche.

De manière avantageuse, l'ingrédient laitier l'invention est présenté sous forme solide et est obtenu par un séchage d'un sérum de crème concentrée provenant d'une concentration thermique et d'un séchage, de préférence par atomisation.

Selon une seconde forme d'exécution préférée de l'invention représentée à la figure 2, 133 kg de sérum de crème concentrée (A5) sont soumis à une étape d'extraction des protéines (« déprotéinisation ») par traitement dit « thermo-calcique » comprenant l'ajout de 0,1% (en poids) de chlorure de calcium , suivi d'un chauffage à une température d'environ 70°C, d'un ajustement du pH du sérum à environ 5.2 par adjonction d'acide citrique et un maintien du sérum à cette température pour une durée d'environ 40 minutes.

Ensuite, les protéines précipitées sont extraites par une étape supplémentaire de décantation centrifuge (séparateur solide-liquide). On obtient ainsi un culot d'environ 21 kg (20,76 kg) comprenant les protéines de sérum de crème concentrée (C2) et un surnageant d'environ 113 kg (113,20 kg) de sérum déprotéiné de crème concentrée (C1).

Celui-ci est ensuite soumis à une ultrafiltration/diafiltration, avec dilution au moyen d'environ 49 kg (48,6 kg) d'eau. Cette opération fournit 16 kg de rétentat constitué d'un sérum déprotéiné, ultrafiltré et diafiltré de crème concentrée (C3) et environ 146 kg (145,80 kg) d'un perméat dilué de sérum déprotéiné de crème concentrée (C4).

Le rétentat est soumis à une étape supplémentaire de séchage (par atomisation) pour obtenir environ 2,2 kg (2,19 kg) de poudre de sérum déprotéiné, ultrafiltré et diafiltré de crème concentrée (C5). Les caractéristiques des produits obtenus sont reprises sur le tableau 2. Le sérum déprotéiné, ultrafiltré et diafiltré de crème concentrée constitue un ingrédient laitier fortement enrichi en lipides polaires du lait ; sa teneur en phospholipides est de plus de 35% en poids, ce pourcentage étant exprimé par rapport à la matière sèche.

Selon une troisième forme d'exécution préférée de l'invention représentée à la figure 3a, le sérum de crème concentrée (A5) (133 kg) peut être combiné à de la poudre de sérum concentrée (A6) (13,4 kg) pour obtenir environ 146 kg (146,4 kg) d'un sérum de crème concentrée (C6) à plus haute teneur en matière sèche.

Ce produit est soumis à une étape de déprotéinisation telle que décrite ci-dessus pour obtenir un surnageant d'environ 108 kg (108,3 kg) constitué d'un sérum concentré et déprotéiné de crème concentrée (C7) et d'un culot d'environ 39 kg (39,05 kg) constitué de protéines de sérum concentré d'une crème concentrée (C8).

Le surnageant est ensuite soumis à une étape d'ultrafiltration/diafiltration avec addition d'environ 77 kg d'eau (77,3 kg) pour obtenir environ 31 kg d'un rétentat constitué de sérum concentré, déprotéiné ultrafiltré et diafiltré de crème concentrée (C9) et environ 154 kg (154,6 kg) d'un permeat dilué d'un sérum concentré, déprotéiné de crème concentrée (C10). Le rétentat peut être soumis avantageusement à une étape supplémentaire de séchage par atomisation pour obtenir un peu plus de 4 kilos (4,25 kg) d'une poudre de sérum concentré, déprotéiné, ultrafiltré et diafiltré de crème concentrée (C11). Les caractéristiques des produits obtenus sont représentées dans le tableau 3. A nouveau, le sérum concentré, déprotéiné, ultrafiltré et diafiltré de crème concentrée obtenu constitue un ingrédient laitier fortement enrichi en lipides polaires du lait ; sa teneur en phospholipides est de plus de 35% en poids, ce pourcentage étant exprimé par rapport à la matière sèche. L'augmentation de la matière sèche du sérum de crème concentrée n'a pas affecté le facteur de concentration des lipides polaires récupérés après déprotéinisation et ultrafiltration/diafiltration.

De manière alternative, il est également possible de traiter la poudre de sérum ultrafiltré/diafiltré de crème concentré (A13) (9,38 kg) par addition d'eau (90,62 kg) pour obtenir 100 kg d'un sérum ultrafiltré/diafiltré de crème concentrée à 9 % de matière sèche (C12). Ce produit est soumis à une étape de déprotéinisation (figure 3b) telle que décrite ci-dessus, pour obtenir 76 kg d'un surnageant constitué d'un sérum ultrafiltré, diafiltré et déprotéiné de crème concentrée (C13) et 24 kg d'un culot constitué de protéines de sérum ultrafiltré, diafiltré de crème concentrée (C14). Les caractéristiques de ces derniers produits sont représentées dans le tableau 4. Le sérum ultrafiltré, diafiltré et déprotéiné de crème concentrée (C13) constitue l'ingrédient laitier concentré en lipides polaires de ce procédé ; sa teneur en phospholipides laitiers est de 0,95%, soit plus de 20% en poids exprimé sur la matière sèche.

### Exemple 2

Tel que représenté à la figure 4a, il est possible d'obtenir également l'ingrédient laitier de l'invention, selon une variante d'exécution de celle représentée à l'exemple 1. Environ 1000 kg de crème de lait pasteurisée (B1) sont soumis à un traitement thermique, similaire à l'exemple 1 et ensuite à une première centrifugation, similaire à celle de l'exemple 1, pour obtenir une phase légère de crème concentrée (B2) et une phase lourde de lait écrémé (B3). Les proportions sont équivalentes à celles de l'exemple 1. La phase légère (B2) est diluée par une quantité similaire d'eau pure (non tamponnée) pour obtenir une crème concentrée diluée (B4) (environ 1000 kg). Cette crème concentrée diluée (B4) est soumise à une seconde concentration par centrifugation de manière à obtenir une phase légère constituée d'une crème concentrée et lavée (B5) et d'une phase lourde constituée d'un lait écrémé dilué (B6) (environ 537 kg de crème concentrée et lavée (B5) et environ 463 kg de lait écrémé dilué (B6)).

La crème concentrée et lavée (B6) est ensuite soumise à une étape d'inversion de l'émulsion et à une nouvelle concentration par centrifugation, telles que décrite dans l'exemple 1. On obtient ainsi à partir de la crème concentrée et lavée (B5) une phase légère constituée d'une matière grasse de lait (B7) (environ 396 kg) et une phase lourde, constituée d'un sérum de crème concentrée et lavée (B8) (environ 140 kg). Ce sérum de crème concentrée et lavée peut être soumis à une étape de séchage, telle que décrite dans l'exemple 1 pour obtenir une poudre de sérum de crème concentrée et lavée (B9) pour un poids total d'environ 4,50 kg.

Ledit sérum de crème concentrée et lavée (B8) peut être à nouveau soumis à une étape d'ultrafiltration, présentée à la figure 4b et telle que déjà décrite pour l'exemple 1, de manière à obtenir environ 21,50 kg de rétentat constitué d'un sérum ultrafiltré de crème concentrée et lavée (B10) et environ 118 kg de perméat de sérum de crème concentrée et lavée (B11). Le rétentat (sérum ultrafiltré de crème concentrée et lavée (B10)) peut être également soumis à une nouvelle étape de séchage, tel que décrit dans l'exemple 1 pour obtenir un produit sous forme solide constitué d'une poudre de sérum ultrafiltré de crème concentrée et lavée (B12) d'un poids total d'environ 3,00 kg.

Le tableau 5 présente les caractéristiques des compositions des produits obtenus selon le procédé de l'exemple 2 illustré dans les figures 4a et 4b. Les paramètres analysés sont les mêmes que ceux présentés dans le tableau 1. Le sérum de crème concentrée et lavée (B8) et le sérum ultrafiltré de crème concentrée et lavée (B10) sont des ingrédients laitiers fortement enrichis en lipides polaires de la membrane des globules gras du lait. La teneur en phospholipides laitiers de ces produits est respectivement de 0,62% et 3,90%, soit respectivement plus de 19% et plus de 27% exprimés sur la matière sèche. La concentration en lipides polaires est réalisée au détriment de l'extrait sec dégraissé pour l'opération de lavage de la crème et au détriment de l'extrait sec dégraissé non protéique pour l'ultrafiltration.

De manière avantageuse, les produits suivant, obtenus lors des procédés décrits ci-dessus dans les exemples 1 et 2 :
- sérum de crème concentrée,
- sérum ultrafiltré et éventuellement diafiltré de crème concentrée,
- sérum déprotéiné, ultrafiltré et éventuellement diafiltré de crème concentrée,
- sérum de crème concentrée et lavée, et
- sérum ultrafiltré de crème concentrée et lavée,
enrichi en lipides polaires de la membrane des globules gras du lait (phospholipides et sphingolipides), sous leur forme liquide et/ou éventuellement sous leur forme solide, peuvent être utilisés dans une composition alimentaire et être incorporés avec les composants alimentaires habituels d'une composition alimentaire destinée à l'humain ou à l'animal.

Il est ainsi possible d'obtenir des compositions alimentaires (éventuellement allégées en certaines matières grasses, néfastes pour la santé, en particulier allégées en lipides non polaires), tout en maintenant les propriétés organoleptiques des produits standards ou en offrant des propriétés organoleptiques améliorées par rapport aux produits standards.

L'ingrédient alimentaire de l'invention, caractérisé par un effet émulsifiant, peut être utilisé pour améliorer l'onctuosité des produits alimentaires, notamment des produits laitiers (allégés ou non en matières grasses), tels que des crèmes, des yoghourts, des yoghourts à boire, des fromages, en particulier des fromages à tartiner ou des desserts laitiers.

En outre, l'ingrédient alimentaire permet une meilleure rétention d'eau dans les produits de boulangerie/pâtisserie (pains au lait et brioches) améliorant ainsi la conservation du moelleux de ces produits par rapport à des produits standard préparés sans cet ingrédient alimentaire. En effet, les inventeurs ont observé de manière inattendue une amélioration de la rétention de l'eau dans les produits obtenus ce qui permet une meilleure conservation du moelleux des produits en diminuant leur séchage.

L'ingrédient laitier de l'invention peut être également utilisé pour améliorer l'aptitude au fouettage d'une crème laitière entière et allégée ayant ou non subi un traitement thermique de stérilisation UHT et à laquelle l'ingrédient laitier de l'invention a été incorporé.
On observe également un effet émulsifiant naturel du lait favorisant les émulsions de type « huile dans eau » obtenu avec le produit sous forme de poudre dispersée dans de l'eau ou avec le produit sérum liquide de l'invention.

Les inventeurs ont mis en évidence que des crèmes à 5 % de matière grasse, reconstituée au départ de matière grasse du lait, de poudre de lait écrémé, d'eau et soit, de poudre de sérum de crème concentrée (A6) d'une part ou des extraits riches en phospholipides (C5 : Poudre de sérum déprotéiné, ultrafiltré et diafiltré de crème concentrée ou C11 : Poudre de sérum concentré, déprotéiné, ultrafiltré et diafiltré de crème concentrée) d'autre part, ne présentaient pas la même stabilité en raison de la différence de taille naturelle de leur globules gras. Pour une teneur variant de 0,3 à 1,5% de poudre de sérum de crème concentrée (A6), la taille moyenne des globules gras évoluait respectivement de 5 vers 3 µm. Lorsque la source de phospholipides était représentée par les extraits C5 (Poudre de sérum déprotéiné, ultrafiltré et diafiltré de crème concentrée) ou C11 (Poudre de sérum concentré, déprotéiné, ultrafiltré et diafiltré de crème concentrée) dans les mêmes concentrations (0,3 à 1,5%), la taille moyenne des globules gras évoluait respectivement de environ 2,5 à environ 1 µm.

En outre, l'ingrédient laitier de l'invention présente une concentration élevée en lipides polaires, en particulier en phospholipides et en sphingolipides permettant d'améliorer de manière significative l'état de santé d'un patient, ou le maintien de l'état de santé du patient consommant directement cet ingrédient ou un produit alimentaire contenant cet ingrédient (H. Vesper et al. American Society for Nutritional Sciences, p. 1239-1250 (1999)).

Cet ingrédient enrichi en sphingolipides (sphingomyéline,...) permet avantageusement d'obtenir une réduction du taux de cholestérol sanguin VLDL et LDL et des triglycérides, un effet préventif du cancer, en particulier du cancer du côlon, permet de renforcer l'immunité et la flore intestinale, en particulier de provoquer un effet prébiotique, c'est-à-dire de favoriser la croissance d'une flore intestinale bénéfique (germes de type Bifidus notamment), par rapport à une flore intestinale pathogène, et de prévenir ou traiter des troubles digestifs (diarrhées).

En outre, l'ultra concentration dans l'ingrédient laitier des phospholipides et des sphingolipides peut avoir des effets anti-diabétiques (traitement et/ou prévention du diabète) et assurer la protection du foie.

Un dernier aspect de l'invention concerne une composition cosmétique ou une composition pharmaceutique (alicament ou « functional food ») comprenant un véhicule pharmaceutique adéquat et l'ingrédient laitier de l'invention, en particulier destiné au traitement et/ou à la prévention des pathologies susmentionnées, ainsi qu'un procédé de traitement préventif ou thérapeutique de l'une des pathologies susmentionnées chez un mammifère (y compris l'homme) dans lequel une quantité suffisante de cette composition est administrée à un mammifère (y compris l'homme) susceptible de souffrir de ces pathologies.

**Tableau 1**

| % en poids | Eau | Matière grasse totale | dont Phospholipides | Extrait sec dégraissé | dont Protéines | dont Lactose | dont Cendres |
|---|---|---|---|---|---|---|---|
| (A1) Crème de lait pasteurisée | 54.90% | 40.00% | 0.22% | 5.10% | 1.83% | 2.84% | 0.42% |
| (A2) Crème de lait concentrée | 22.88% | 75.00% | 0.35% | 2.13% | 0.76% | 1.18% | 0.18% |
| (A3) Lait écrémé | 91.13% | 0.40% | 0.07% | 8.47% | 3.04% | 4.72% | 0.71% |
| (A4) Matière grasse de lait | 0.37% | 99.60% | 0.23% | 0.03% | 0.01% | 0.02% | 0.00% |
| (A5) Sérum de crème concentrée | 90.17% | 1.45% | 0.75% | 8.38% | 3.01% | 4.67% | 0.70% |
| (A6) Poudre de sérum de crème concentrée | 4.00% | 14.17% | 7.33% | 81.83% | 29.40% | 45.62% | 6.82% |
| (A7) Sérum ultrafiltré de crème concentrée | 82.69% | 4.24% | 2.10% | 13.07% | 8.30% | 3.60% | 1.17% |
| (A9) Poudre de sérum ultrafiltré de crème concentrée | 4.00% | 23.51% | 11.65% | 72.49% | 46.03% | 19.97% | 6.49% |
| (A11) Sérum ultrafiltré et diafiltré de crème concentrée | 82.46% | 5.00% | 2.75% | 12.54% | 10.70% | 0.70% | 1.14% |
| (A13) Poudre de sérum ultrafiltré et diafiltré de crème concentrée | 4.00% | 27.40% | 15.00% | 68.60% | 58.53% | 3.83% | 6.24% |

**Tableau 2**

| % en poids | Eau | Matière grasse totale | dont Phospholipides | Extrait sec dégraissé | dont Protéines | dont Lactose | dont Cendres |
|---|---|---|---|---|---|---|---|
| (A5) Sérum de crème concentrée | 90.17% | 1.45% | 0.75% | 8.38% | 3.01% | 4.67% | 0.70% |
| (C1) Sérum déprotéiné de crème concentrée | 92.08% | 1.43% | 0.78% | 6.49% | 0.90% | 4.73% | 0.86% |
| (C2) Protéines de sérum de crème concentrée | 79.62% | 1.45% | 0.72% | 18.93% | 14.00% | 4.10% | 0.83% |
| (C3) Sérum déprotéiné, ultrafiltré et diafiltré de crème concentrée | 86.80% | 8.70% | 5.20% | 4.50% | 3.60% | 0.65% | 0.25% |
| (C4) Perméat dilué de sérum déprotéiné de crème concentrée | 95.47% | <0.10% | <0.10% | 4.50% | 0.25% | 3.60% | 0.65% |
| (C5) Poudre de sérum déprotéiné, ultrafiltré et diafiltré de crème concentrée | 3.50% | 63.60% | 38.00% | 32.90% | 26.30% | 4.76% | 1.84% |

**Tableau 3**

| % en poids | Eau | M atière grasse totale | dont Phospholipides | Extrait sec dégraissé | dont Protéines | dont Lactose | dont Cendres |
|---|---|---|---|---|---|---|---|
| (C6) Sérum concentré de crème concentrée | 82.30% | 2.60% | 1.35% | 15.10% | 5.30% | 8.51% | 1.29% |
| (C7) Sérum concentré et déprotéiné de crème concentrée | 85.90% | 2.60% | 1.45% | 11.50% | 1.35% | 8.75% | 1.40% |
| (C8) Protéines de sérum concentré de crème concentrée | 72.20% | 2.50% | 1.00% | 25.30% | 16.00% | 7.90% | 1.40% |
| (C9) Sérum concentré, déprotéiné, ultrafiltré et diafiltré de crème concentrée | 86.75% | 8.80% | 5.30% | 4.45% | 3.60% | 0.60% | 0.25% |
| (C10) Perméat dilué de sérum concentré, déprotéiné de crème concentrée | 92.83 | < 0.10% | <0.10% | 7.15% | 0.25% | 6.00% | 0.90% |
| (C11) Poudre de sérum concentré, déprotéiné, ultrafiltré et diafiltré de crème concentrée | 3.00% | 64.42% | 38.80% | 32.58% | 26.35% | 4.39% | 1.83% |

**Tableau 4**

| % en poids | Eau 4.00% | Matière grasse totale | dont Phospholipides | Extrait sec dégraissé | dont Protéines | dont Lactose | dont Cendres |
|---|---|---|---|---|---|---|---|
| (A13) Poudre de sérum ultrafiltré et diafiltré de crème concentrée | | 27.40% | 15.00% | 68.60% | 58.53% | 3.83% | 6.24% |
| (C12) Sérum ultrafiltré et diafiltré de crème concentrée | 91.00% | 2.57% | 1.40% | 6.43% | 5.48% | 0.36% | 0.59% |
| (C13) Sérum ultrafiltré, diafiltré et déprotéiné de crème concentrée à 9% matière sèche | 95.58% | 1.85% | 0.95% | 2.57% | 1.59% | 0.38% | 0.60% |
| (C14) Protéines de sérum ultrafiltré et diafiltré de crème concentrée | 76.75% | 4.30% | 2.70% | 18.95% | 18.00% | 0.35% | 0.60% |

**Tableau 5**

| % en poids | Eau | Matière grasse totale | dont Phospholipides | Extrait sec dégraissé | dont Protéines | dont Lactose | dont Cendres |
|---|---|---|---|---|---|---|---|
| (B1) Crème de lait pasteurisée | 54.90% | 40.00% | 0.22% | 5.10% | 1.83% | 2.84% | 0.42% |
| (B2) Crème de lait concentrée | 22.88% | 75.00% | 0.35% | 2.13% | 0.76% | 1.18% | 0.18% |
| (B3) Lait écrémé | 91.13% | 0.40% | 0.07% | 8.47% | 3.04% | 4.72% | 0.71% |
| (B4) Crème concentrée diluée | 59.09% | 39.79% | 0.19% | 1.13% | 0.40% | 0.63% | 0.09% |
| (B5) Crème concentrée et lavée | 25.52% | 74.00% | 0.32% | 0.48% | 0.17% | 0.27% | 0.04% |
| (B6) Lait écrémé dilué | 98.05% | 0.15% | 0.03% | 1.80% | 0.65% | 1.00% | 0.15% |
| (B7) Matière grasse de lait | 0.39% | 99.60% | 0.22% | 0.01% | 0.00% | 0.01% | 0.00% |
| (B8) Sérum de crème concentrée et lavée | 96.90% | 1.30% | 0.62% | 1.80% | 0.65% | 1.00% | 0.15% |
| (B9) Poudre de sérum de crème concentrée et lavée | 4.00% | 40.26% | 19.20% | 55.74% | 20.02% | 31.07% | 4.64% |
| (B10) Sérum ultrafiltré de crème concentrée et lavée | 86.50% | 8.00% | 3.90% | 5.50% | 4.30% | 0.60% | 0.60% |
| (B12) Poudre de sérum ultrafiltré de crème concentrée et lavée | 4.00% | 56.89% | 27.73% | 39.11% | 30.58% | 4.27% | 4.27% |

## Revendications

1. Un procédé d'obtention d'un ingrédient laitier enrichi en lipides polaires, en particulier en phospholipides et en sphingolipides, et dont le pourcentage en phospholipides est supérieur à 36% en poids par rapport au poids de matière sèche de l'ingrédient, et comprenant les étapes de:
- chauffage d'une crème pasteurisée de lait (A1) à une température comprise entre 65°C et 70°C pendant une durée comprise entre 5 et 20 minutes,
- centrifugation d'une crème pasteurisée de lait (A1) en une phase légère d'une crème concentrée (A2) et en une phase lourde de lait écrémé (A3),
- inversion de la phase légère de crème concentrée (A2) en une émulsion eau dans huile,
- séparation par centrifugation de la phase légère de crème concentrée (A2) en une phase légère de matière grasse de lait (A4) et en une phase lourde de sérum de crème concentrée (A5) dans une proportion entre la phase légère de matière grasse de lait (A4) et la phase lourde de sérum de crème concentrée (A5) qui est comprise entre ¾ et ¼, suivie
soit par les étapes de :
- extraction des protéines de la phase lourde de sérum de crème concentrée (A5) par un traitement thermo-calcique comprenant l'ajout de chlorure de calcium, suivi d'un chauffage à une température de 70°C, d'un ajustement du pH du sérum à 5.2 par adjonction d'acide citrique, d'acide phosphorique ou un mélange d'entre eux et un maintien du sérum à cette température pour une durée de 40 minutes et décantation centrifuge pour former un culot de protéines de sérum de crème concentrée (C2) et un surnageant de sérum déprotéiné de crème concentrée (C1).
- ultrafiltration et diafiltration avec dilution par de l'eau pour former un rétentat de sérum déprotéiné, ultrafiltré et diafiltré de crème concentrée (C3) et un perméat dilué de sérum déprotéiné de crème concentrée (C4),
- récupération de l'ingrédient laitier constitué du rétentat de sérum déprotéiné, ulrafiltré et diafiltré de crème concentrée (C3), dont le pourcentage en phospholipides est supérieur à 36% en poids par rapport au poids de matière sèche de l'ingrédient,
ou soit par les étapes de :
- séchage par atomisation de la phase lourde de sérum de crème concentrée (A5) en une poudre de crème concentrée (A6),
- combinaison de la phase lourde de sérum de crème concentrée (A5) avec la poudre de crème concentrée (A6) pour obtenir un sérum concentré de crème concentrée (C6),
- extraction des protéines du sérum concentré de crème concentrée (C6) par un traitement thermocalcique comprenant l'ajout de chlorure de calcium, suivi d'un chauffage à une température de 70°c, d'un ajustement du pH du sérum à 5.2 par adjonction d'acide citrique, d'acide phosphorique ou mélange d'entre eux et un maintien du sérum à cette température pour une durée de 40 minutes et décantation centrifuge pour former un culot de protéines de sérum concentré de crème concentrée (C8) et un surnageant de sérum concentré et déprotéiné de crème concentrée (C7),
- ultrafiltration et diafiltration avec dilution du surnageant de sérum concentré et deprotéiné de crème concentrée (C7) par de l'eau pour obtenir un rétentat de sérum concentré, déprotéiné, ultrafiltré et diafiltré de crème concentrée (C9) et un perméat dilué de sérum concentré et déprotéiné de crème concentrée (C10) et
- récupération d'un ingrédient laitier constitué du rétentat de sérum concentré, déprotéiné, ultrafiltré et diafiltré de crème concentrée (C9) et dont le pourcentage en phospholipides est supérieur à 36% en poids par rapport au poids en matière sèche de l'ingrédient.

2. Le procédé selon la revendication 1, comprenant en outre un séchage par atomisation
- du rétentat de sérum déprotéiné, ultrafiltré et diafiltré de crème concentrée (C3) en une poudre de sérum déprotéiné, ultraflitré et diafiltré de crème concentrée (C5) ou
- du rétentat de sérum concentré, déprotéiné, ultrafiltré et diafiltré de crème concentrée (C9) en une poudre de sérum concentré, déprotéiné, ultrafiltré et diafiltré de crème concentrée (C11).

3. Le procédé selon la revendication 1 ou la revendication 2, dans lequel le pourcentage en phospholipides est de 38% en poids par rapport au poids de matière sèche de l'ingrédient.

4. Le procédé selon l'une quelconque des revendications précédentes 1 à 3, dans lequel le pourcentage en phospholipides est de 38,8% en poids par rapport au poids de matière sèche de l'ingrédient.

## Patentansprüche

1. Ein Verfahren zur Herstellung eines mit polaren Lipiden, insbesondere mit Phospholipiden und mit Sphingolipiden, angereicherten Molkereibestandteils, und dessen Prozentsatz an Phospholipiden über 36 Gew.-% in Bezug auf das Trockenmassegewicht des Bestandteils beträgt, und umfassend die folgenden Schritte:
- Erhitzen einer pasteurisierten Milchcreme (A1) auf eine Temperatur zwischen 65 °C und 70 °C während einer Dauer zwischen 5 und 20 Minuten,
- Zentrifugieren einer pasteurisierten Milchcreme (A1) in eine leichte Cremekonzentratphase (A2) und in eine schwere Phase entrahmter Milch (A3),
- Invertieren der leichten Cremekonzentratphase (A2) in eine Wasser-in-ÖI-Emulsion,
- Zentrifugalseparation der leichten Cremekonzentratphase (A2) in eine leichte Milchfettphase (A4) und in eine schwere Cremekonzentratserumphase (A5) in einem Verhältnis zwischen der leichten Milchfettphase (A4) und der schweren Cremekonzentratserumphase (A5) das zwischen ¾ und ¼ liegt, gefolgt
entweder von den Schritten:
- Extrahieren der Proteine der schweren Cremekonzentratserumphase (A5) durch eine Thermo-Kalzium-Behandlung, umfassend das Hinzufügen von Calciumchlorid, gefolgt von einem Erwärmen auf eine Temperatur von 70 °C, einer Korrektur des pH des Serums auf 5,2 durch Zusetzen von Citronensäure, Phosphorsäure oder eines Gemischs beider und einem Halten des Serums auf dieser Temperatur für eine Dauer von 40 Minuten und Zentrifugaldekantieren, um eine Schicht aus Cremekonzentratserumproteinen (C2) und einen Überstand aus proteinfreiem Cremekonzentratserum (C1) zu bilden.
- Ultrafiltrieren und Diafiltrieren mit Verdünnung durch Wasser, um ein ultrafiltriertes und diafiltriertes proteinfreies Cremekonzentratserumretentat (C3) und ein verdünntes proteinfreies Cremekonzentratserumpermeat (C4) zu bilden,
- Rückgewinnen des Molkereibestandteils, bestehend aus dem ultrafiltrierten und diafiltrierten proteinfreien Cremekonzentratserumretentat (C3), dessen Prozentsatz an Phospholipiden über 36 Gew.-% in Bezug auf das Trockenmassegewicht des Bestandteils beträgt,
oder durch die Schritte:
- Sprühtrocknen der schweren Cremekonzentratserumphase (A5) in ein Cremekonzentratpulver (A6),
- Kombinieren der schweren Cremekonzentratserumphase (A5) mit dem Cremekonzentratpulver (A6), um ein konzentriertes Cremekonzentratserum (C6) zu erhalten,
- Extrahieren der Proteine des konzentrierten Cremekonzentratserums (C6) durch eine Thermo-Kalzium-Behandlung, umfassend das Hinzufügen von Calciumchlorid, gefolgt von einem Erwärmen auf eine Temperatur von 70 °C, einer Korrektur des pH des Serums auf 5,2 durch Zusetzen von Citronensäure, Phosphorsäure oder eines Gemischs beider und einem Halten des Serums auf dieser Temperatur für eine Dauer von 40 Minuten und Zentrifugaldekantieren, um eine Schicht aus konzentrierten Cremekonzentratserumproteinen (C8) und einen Überstand aus konzentriertem und proteinfreiem Cremekonzentratserum (C7) zu bilden,
- Ultrafiltrieren und Diafiltrieren mit Verdünnung des Überstands aus konzentriertem und proteinfreiem Cremekonzentratserum (C7) durch Wasser, um ein ultrafiltriertes und diafiltriertes proteinfreies konzentriertes Cremekonzentratserumretentat (C9) und ein verdünntes konzentriertes und proteinfreies Cremekonzentratserumpermeat (C10) zu bilden, und
- Rückgewinnen eines Molkereibestandteils, bestehend aus dem ultrafiltrierten und diafiltrierten, konzentrierten, proteinfreien Cremekonzentratserumretentat (C9), und dessen Prozentsatz an Phospholipiden über 36 Gew.-% in Bezug auf das Trockenmassegewicht des Bestandteils beträgt.

2. Das Verfahren nach Anspruch 1, umfassend ferner eine Sprühtrocknung
- des ultrafiltrierten und diafiltrierten proteinfreien Cremekonzentratserumretentats (C3) in ein ultrafiltriertes und diafiltriertes proteinfreies Cremekonzentratserumpulver (C5) oder
- des ultrafiltrierten und diafiltrierten proteinfreien Cremekonzentratserumretentats (C9) in ein ultrafiltriertes und diafiltriertes proteinfreies konzentriertes Cremekonzentratserumpulver (C11).

3. Das Verfahren nach Anspruch 1 oder Anspruch 2, wobei der Prozentsatz an Phospholipiden 38 Gew.-% in Bezug auf das Trockenmassegewicht des Bestandteils beträgt.

4. Das Verfahren nach einem der vorangehenden Ansprüche 1 bis 3, wobei der Prozentsatz an Phospholipiden 38,8 Gew.-% in Bezug auf das Trockenmassegewicht des Bestandteils beträgt.

## Claims

1. A method for obtaining a dairy ingredient enriched in polar lipids, in particular in phospholipids and sphingolipids, and wherein the percentage of phospholipids is greater than 36% by weight relative to the weight of dry matter of the ingredient, and comprising the steps of:
- heating of a pasteurized milk cream (A1) to a temperature between 65°C and 70°C for a period of between 5 and 20 minutes,
- centrifugation of a pasteurized milk cream (A1) in a light phase of a concentrated cream (A2) and in a heavy phase of skimmed milk (A3),
- inversion of the light phase of concentrated cream (A2) in a water-in-oil emulsion,
- separation by centrifugation of the light phase of concentrated cream (A2) into a light phase of milk fat (A4) and a heavy phase of concentrated cream serum (A5) in a proportion between the light phase of milk fat (A4) and the heavy phase of concentrated cream serum (A5) which lies between 3/4 and 1/4, followed
either by the steps of:
- extraction of the proteins from the heavy phase of concentrated cream serum (A5) by a thermo-calcic treatment comprising the addition of calcium chloride, followed by heating to a temperature of 70°C, by adjusting the pH of the serum to 5.2 by adding citric acid, phosphoric acid or a mixture of both, while maintaining the serum at this temperature for a period of 40 minutes, and by centrifugal decantation to form a pellet of proteins of concentrated cream serum (C2) and a supernatant of deproteinized concentrated cream serum (C1).
- ultrafiltration and diafiltration with dilution with water to form a retentate of deproteinized, ultrafiltered and diafiltered concentrated cream serum (C3), and a diluted permeate of deproteinized concentrated cream serum (C4),
- recovery of the dairy ingredient comprising the retentate of deproteinized, ultrafiltered and diafiltered concentrated cream serum (C3), of which the percentage of phospholipids is greater than 36% by weight relative to the weight of dry matter of the ingredient,
or by the steps of
- spray drying the heavy phase of concentrated cream serum (A5) into a concentrated cream powder (A6),
- combination of the heavy phase of concentrated cream serum (A5) with the concentrated cream powder (A6) to obtain a concentrated cream serum (C6),
- extraction of the proteins from the concentrated cream serum (C6) by a thermo-calcic treatment comprising the addition of calcium chloride, followed by heating to a temperature of 70°C, by adjusting the pH of the serum to 5.2 by adding citric acid, phosphoric acid or a mixture of both and maintaining the serum at this temperature for a period of 40 minutes, and centrifugal decantation to form a pellet of proteins of concentrated serum of concentrated cream (C8), and a supernatant of concentrated and deproteinized serum of concentrated cream (C7),
- ultrafiltration and diafiltration with dilution of the supernatant of concentrated and deproteinized serum of concentrated cream (C7) with water to obtain a retentate of concentrated, deproteinized, ultrafiltrated and diafiltrated serum of concentrated cream (C9), and a diluted permeate of concentrated and deproteinized serum of concentrated cream (C10), and
- recovery of a dairy ingredient comprising the retentate of concentrated, deproteinized, ultrafiltrated and diafiltrated serum of concentrated cream (C9), and of which the percentage of phospholipids is greater than 36% by weight relative to the weight of dry matter of the ingredient.

2. The method according to claim 1, further comprising a spray-drying
- of the retentate of deproteinized, ultrafiltered and diafiltered concentrated cream serum (C3) into a powder of deproteinized, ultrafiltered and diafiltered concentrated cream serum (C5), or
- of the retentate of concentrated, deproteinized, ultrafiltered and diafiltered serum of concentrated cream(C9) into a powder of concentrated, deproteinized, ultrafiltered and diafiltered serum of concentrated cream (C11).

3. The method according to claim 1 or claim 2, wherein the percentage of phospholipids is 38% by weight relative to the weight of dry matter of the ingredient.

4. The method according to any one of the preceding claims 1 to 3, wherein the percentage of phospholipids is 38.8% by weight based on the weight of dry matter of the ingredient.
